# EUROPÄISCHE PATENTSCHRIFT

(11) **EP 2 524 683 B1**
(45) Veröffentlichungstag und Bekanntmachung des Hinweises auf die Patenterteilung: **26.09.2018**
(21) Anmeldenummer: 11195854.2
(22) Anmeldetag: 28.12.2011
(51) Int. Cl.: A61H 39/04, A61H 39/06

(54) **Kinesiologisches Tape**
Kinesio tape
Bande kinésiologique

(30) Priorität: 17.05.2011 DE 202011100806 U; 19.07.2011 DE 202011103469 U; 29.09.2011 DE 102011083681
(43) Veröffentlichungstag der Anmeldung: 21.11.2012
(73) Patentinhaber: Altinok, Gazi, 42859 Remscheid (DE); Vafiadis, Stefanos, 58093 Hagen (DE); Zemichael, Tesfaldet, 44135 Dortmund (DE); Celik, Abidin, 42897 Remscheid (DE)
(72) Erfinder: Altinok, Gazi, 42859 Remscheid (DE); Vafiadis, Stefanos, 58093 Hagen (DE); Zemichael, Tesfaldet, 44135 Dortmund (DE); Celik, Abidin, 42897 Remscheid (DE)
(74) Vertreter: Gille Hrabal

(56) Entgegenhaltungen:
- WO-A2-2007/084660
- DE-U1- 7 934 840
- DE-U1- 8 319 476
- DE-U1- 8 319 476
- DE-U1-202009 014 424
- JP-A- 2011 087 922
- JP-A- 2012 029 983
- US-A- 4 162 672
- US-A1- 2009 222 072

## Beschreibung

Die vorliegende Erfindung betrifft ein kinesiologisches Tape, welches mit mindestens einem Formkörper ausgestattet ist, wobei dieser Formkörper Kupfer umfasst.

Kinesiologische Tcpes sind insbesondere in der Sportmedizin und Orthopädie bekannt. Sie stellen elastische Pflaster dar, deren Elastizitätseigenschaften denen des menschlichen Muskels angepasst sind. Dadurch resultiert keine Bewegungseinschränkungen des Patienten. Kinesiologische Tapes nehmen gezielt Einfluss auf das Nerven- und Stoffwechselsystem des Patienten, Durch die Anwendung unterschiedlicher Klebetechniken erreicht der Therapeut zum Beispiel bei Prellungen, überstrapazierten Gelenken oder Muskelproblemen eine deutlich spürbare und vor allern schnelle Schmerzreduktion. Des Weiteren kommt es aber auch zu einer nachhaltigen Verbesserung der Muskelfunktion und Unterstützung der Gelenke. Durch das Tape kommt es zu einer gezielten Anhebung bestimmter Hautpartien, wodurch zusätzlich auch das Lymphsystem stimuliert wird. Auch positive Auswirkungen auf innere Organe wurden bereits beschrieben. Hierbei werden vor allem unterschiedliche Anlegetechniken in Kombination mit der Vordehnung des Tapes genutzt,

Bei den gängigen Klebetechniken kommt es vor allem zur Reizübertragung über das beklebte Gewebe. Diese Reize sind auf Grund der Beschaffenheit des Tapes meistens flächig ausgeprägt und daher nicht gezielt auf eine einzige Muskelpartie beschränkt.

Die klassische Akupunktur verwendet Nadeln in bestimmten, dem jeweiligen Organ oder Nerv zugeordneten Hautstellen, die bis in die Unterhaut eingestochen werden, wodurch eine Beruhigung, Kräftigung und Wiederherstellung der Funktion des betroffenen Organs oder Nervs erzielt wird. Es gilt jedoch als erwiesen, dass auch die Akupressur bei der Behandlung von Krankheiten bzw, Schmerzen ebenso gezielt eingesetzt werden kann. Hier wird ein äußerer - meist auch gezielt punktueller - Druck auf den Körper gerichtet, der eine heilende Beeinflussung bestimmter Areale zur Folge hat. Kommt es zu einer gezielt punktuellen Druckausübung, so bezeichnet man den kraftbeaufschlagten Bereich als Reflexpunkt. Die Akupressur erfordert jedoch einen steten Druck von außen, der auf den Körper gerichtet wird. Daher sind längere manuelle Akupressurbehandlungen für den Therapeuten meist sehr ermüdend.

Das Dokument DE202009014424U1 beschreibt ein kinesiologisches Tape.

Die DE 42 40 952 A1 beschreibt eine Vorrichtung zur gezielten Anwendung der Akupressur, mit der über einen längeren Zeitraum hinweg eine punktuelle Kraftbeaufschlagung erfolgen kann. Dazu verwendet sie einen Verband, der so ausgebildet ist, dass er an vorgegebenen Stellen Erhebungen bzw. Noppen oder Warzen aufweist. Diese bestehen aus Kunststoff oder einem hochverdichteten Textilgewebe. Ebenso beschreibt die DE 7 934 840 U1 eine Vorrichtung zur Zonentherapie, wobei ein wundpflasterartiger Träger mit einem nichtdehnbaren Bereich, auf dem wiederum eine harte Erhebung aufgebracht ist, verwendet wird. Auch in der EP 0 779 068 A1 wird eine gesundheitsfördernde Vorrichtung mit Akupressureffekt beschrieben, die aus einem Träger mit Erhebungen besteht. Dabei werden die Erhebungen aus Silikon mit geeigneter Elastizität gebildet.

Im Gegensatz dazu beschreibt die EP 0 081 109 A2 ein Heilpflaster für die Behandlung größerer Flächen, wobei Dauermagnete auf dieses Pflaster aufgebracht werden. Sie werden so in das Pflaster eingebettet, dass eine drucklose Aufbringung auf die Haut ermöglicht wird.

So ist einerseits aus dem Stand der Technik ein kinesiologisches Tape bekannt, welches durch seine Beschaffenheit die Eigenschaften zur Stimulation und Reizausübung flächiger Körperpartien besitzt. Zum anderen ist die Anbringung von Erhebungen unterhalb von meist nicht dehnbaren Trägern zur druckausübenden Einwirkung auf bestimmte Reflexzonen bekannt.

Nachteilig an den bisher bekannten Systemen ist zum einen, dass kinesiologische Tapes nur therapiebegleitend zur Linderung von Schmerzen verwendet werden. Mit diesen Tapes können keine Reflexpunkte angeregt werden.

Die bekannten Akupressurvorrichtungen weisen hingegen den Nachteil auf, dass hier nur punktuelle Reize gesetzt werden. Eine Anregung des gesamten Stoffwechsels durch die Stimulierung des Unterhautgewebes, der Muskelfaszie und des Muskelgewebes ist durch die genannten Akupressurvorrichtungen nicht möglich, Des Weiteren können diese Vorrichtungen nicht individuell auf die betroffene Körperregion und vor allem auch nicht auf den individuellen Patienten angepasst werden.

Darüber hinaus besitzen beide Therapiemethoden den Nachteil, dass eine unterstützende Wärme- oder Kältetherapie der betreffenden Körperteile nur bedingt möglich ist, Die wärmeisolierende Wirkung des kinesiologischen Tapes einerseits aber auch der Erhebungen der Akupressurvorrichtungen andererseits verhindern eine Wärmebeziehungsweise Kälteübertragung zu den beklebten/kraftbeaufschlagten und damit zu therapierenden Regionen.

Ausgehend von diesem Stand der Technik stellt sich die vorliegende Erfindung somit die Aufgabe, ein kinesiologisches Tape bereitzustellen, welches gleichzeitig auch zur Druckausübung mit Akupressurwirkung auf das beklebte Körperteil geeignet ist,

Dabei soll die vorliegende Erfindung vorzugsweise eine erhöhte schmerzlindernde Wirkung und die verbesserte Stimulation der Durchblutung, der Tonusregulation der Muskulatur und des Venös/lymphatischen Rückflusses mit sich bringen.

Darüber hinaus soll die vorliegende Erfindung ein kinesiologisches Tape bereitstellen, durch das vorzugsweise auch eine gleichzeitige punktuelle Druckausübung mit Akupressurwirkung auf das beklebte Körperteil möglich ist.

Ferner soll die Erfindung vorzugsweise eine Anpassung des gegebenenfalls punktuellen Drucks auf die Bedürfnisse des individuellen Patienten ermöglichen. Hier soll vor allem eine Individualisierung auf das jeweilige Krankheitsbild, das dadurch zu therapierende Körperteil und die jeweilige Körperform des einzelnen Patienten möglich sein,

In einem weiteren Aspekt der vorliegenden Erfindung soll das kinesiologische Tape vorzugsweise auch zur unterstützenden Wärmetherapie geeignet sein.

Das kinesiologische Tape soll in einem weiteren Aspekt der vorliegenden Erfindung vorzugsweise auch zur unterstützenden Kältetherapie geeignet sein.

Weiterhin soll das kinesiologische Tape in einem weiteren Aspekt der vorliegenden Erfindung vorzugsweise auch zur unterstützenden Massage durch Vibrationen geeignet sein.

Gelöst werden diese Aufgaben durch die Bereitstellung eines kinesiologischen Tapes mit mindestens einem Formkörper zur Druckausübung mit Akupressurwirkung auf eines beklebte Körperteil, der ein Material umfasst, welches Kupfer ist. Es hat sich gezeigt, dass die Verwendung eines kinesiologischen Tapes in Kombination mit mindestens einem Formkörper besonders gute schmerzstillende Eigenschaften besitzt und positive Auswirkungen in Bezug auf die Heilung des Patienten zu beobachten sind. Überraschenderweise konnte festgestellt werden, dass eine schmerzlindernde Wirkung des Tapes auf der einen Seite und eine heilende Akupressurwirkung auf der anderen Seite überadditiv durch das erfindungsgemäße kinesiologische Tape übertroffen werden kann.

In einem zweiten Aspekt der vorliegenden Erfindung besteht der mindestens eine Formkörper aus Kupfer.

In einem weiteren Aspekt der vorliegenden Erfindung ist der mindestens eine Formkörper auf der zu beklebenden Körperseite des kinesiologischen Tapes aufgebracht,

In einem weiteren Aspekt ist der mindestens eine Formkörper derart, dass er Druck auf mindestens einen Reflexpunkt ausüben kann. Unter einem Reflexpunkt wird Im Rahmen der vorliegenden Erfindung die Fläche verstanden, die durch einen Formkörper auf einem Körperteil bedeckt wird, Dieser Begriff wird gleichbedeutend für die in der Literatur ebenfalls gängigen Begriffe Reflexzone, Triggerpunkt und Bindegewebszone verwendet. Bevorzugt wird auf diesen Reflexpunkt durch den mindestens einen Formkörper Druck ausgeübt. In einer Ausfürhungsform der vorliegenden Erfindung kann durch die Druckausübung über den mindestens einen Formkörper ein entsprechendes inneres Organ stimuliert werden.

In einer weitere Ausführungsform ist dieser mindestens eine Formkörper derart ausgebildet, dass ein flächiger Druck auf einen Reflexpunkt ausgeübt wird. Dazu weist der mindestens eine Formkörper eine flache Form auf. Diese flache Form kann bevorzugt auch eine Wölbung aufweisen. In einer anderen Ausführungsform ist dieser mindestens eine Formkörper derart ausgebildet, dass ein punktueller Druck auf einen Reflexpunkt ausgeübt wird. Hierbei wird eine spitze Form des mindestens einen Formkörpers bevorzugt.

Unter dem Begriff "mindestens ein Formkörper" wird im Rahmen der vorliegenden Erfindung verstanden, dass ein oder mehrere gleichförmige Formkörper, ein oder mehrere ungleichförmige Formkörper, ein oder mehrere Formkörper aus gleichen Materialien, ein oder mehrere Formkörper aus unterschiedlichen Materialien und/oder Kombinationen davon eingesetzt werden können.

In einer weiteren Ausführungsform weist das kinesiologische Tape zwei oder mehr Formkörper auf, die derart sind, dass gleichzeitig ein punktueller und flächiger Druck auf zwei oder mehr Reflexpunkte ausgeübt wird,

In einem weiteren Aspekt der vorliegenden Erfindung wird ein kinesiologisches Tape mit mindestens einem auf der zu beklebenden Körperseite zugewandten Seite aufgebrachten Formkörper zur druckausübenden Einwirkung auf mindestens einen Reflexpunkt bereitgestellt, wobei der mindestens ein Formörper Kupfer umfasst. Kupfer weist eine hohe Wärmeleitfähigkeit auf. Dadurch ist es besonders geeignet für eine gezielte Wärme- und/oder Kältetherapie. Des Weiteren werden diesem Material Eigenschaften zugesprochen, die die Heilung eines Patienten unterstützen.

Das Material des mindestens einen Formkörpers kann zum Beispiel, das nicht Teil der Erfindung ist, eine hohe Wärmeleitfähigkeit von 240 bis 380 W/(m·K) aufweisen.

Dabei wird bevorzugt ein kinesiologisches Tape mit mindestens einem Formkörper bereitgestellt, wobei der mindestens eine Formkörper eine Form besitzt ausgewählt aus der Gruppe, bestehend aus Kugel, Kugelkette, Ring, Halbkugel, Ellipsoid, Rotationsellipsoid, Paraboloid, Rotationsparaboloid, Hyperboloid, Oloid, Polyeder, Tetraeder, Würfel, Quader, Oktaeder, Ikosaeder, Dodekaeder, Kuboktaeder, Rhombendodekaeder, Ikosidodekaeder, Rhombentriaktontaeder, Deltaeder, Pyramide, Prisma, Antiprisma, Pyramidenstumpf, Torus, Zylinder, Kegel, Kegelstumpf, geodätischer Kuppel und/oder Kombinationen davon, Der mindestens eine Formkörper kann so ausgebildet beziehungsweise auf dem Tape aufgebracht sein, dass dabei gegebenenfalls mindestens eine Fläche des Formkörpers auf mindestens einem Reflexpunkt aufliegt, wobei die Fläche ausgewählt wird aus der Gruppe bestehend aus Rechteck, Quadrat, Dreieck, Raute, Kreis, Ellipse, Oval und/oder Kombinationen davon. In einer bervorzugten Ausführungsform wird ein kreisförmiger Formkörper verwendet, der eine leichte Wölbung aufweist, Die Wölbung zeigt dabei vorzugsweise zum beklebten Reflexpunkt. Durch die so gestaltete Ausführungsform kann vorzugsweise eine besonders gute friktierende Massage des Reflexpunktes realisiert werden.

Das erfindungsgemäße kinesiologisches Tape umfasst in einer bevorzugten Ausführungsform zwei oder mehr Formkörper gleicher und/oder unterschiedlicher Größe. Diese zwei oder mehr Formkörper sind so auf dem Tape angeordnet sind, dass sich eine Dreiecksform, gegebenenfalls mit weiteren Formkörpern gleicher oder unterschiedlicher Größe in der vom Dreieck umschlossenen Fläche, eine Kreisform, gegebenenfalls mit weiteren Formkörpern gleicher oder unterschiedlicher Größe in der vom Kreis umschlossenen Fläche, eine Spiralform, eine Sternform, eine Linie, ein Rechteck, gegebenenfalls mit weiteren Formkörpern gleicher oder unterschiedlicher Größe in der vom Rechteck umschlossenen Fläche, eine Kreuzform, eine X-Form, eine C-Form, eine T-Form, eine L-Form, eine Y-Form, gegebenenfalls mit weiteren Linien, und/oder eine S-Form, gegebenenfalls auch spiegelverkehrt, ergibt und diese Formen gegebenenfalls auch durch mehrere parallel angeordnete Formkörper gebildet und durch die parallele Anordnung verbreitert werden.

Als Alternative, nicht Teil der Erfindung, umfasst das kinesiologisches Tape mindestens einen Formkörper mit einer Höhe von 0,1 bis 5 mm, bevorzugt 0,2 bis 3,5 mm, besonders bevorzugt 0,5 bis 2,5 mm. In einer weiteren Ausführungsform umfasst das kinesiologisches Tape mindestens einen Formkörper mit einer Breite von 0,05 bis 10 cm, bevorzugt 0,1 bis 9 cm, besonders bevorzugt 0,2 bis 8 cm. In einem weiteren Beispiel umfasst das kinesiologisches Tape mindestens einen Formkörper mit einer Länge von 0,05 bis 10 cm, bevorzugt 0,1 bis 9 cm, besonders bevorzugt 0,2 bis 8 cm. Bei runden Formkörpern entspricht die Breite des Formkörpers dem Durchmesser. In einer weiteren Ausführungsform weist der mindestens eine Formkörper eine Wölbung von bis zu 0,5 cm, bevorzugt, 0,25 cm, besonders bevorzugt 0,1 cm Höhe auf.

In einem Beispiel, das nicht Teil der Erfindung ist, ist der mindestens eine Formkörper des kinesiologischen Tapes ein integraler Bestandteil des Tapes. In einem anderen Beispiel, das nicht Teil der Erfindung ist, wird der mindestens eine Formkörper auf das eigentliche kinesiologische Tape aufgesetzt und/oder aufgeklebt.

Das eigentliche kinesiologische Tape umfasst in einem weiteren Beispiel, das nicht Teil der Erfindung ist, ein Material, ausgewählt aus der Gruppe, bestehend aus Bauwollfasern, Polyamid, Nylon, Spandex, Poylester, Elastan, Polyethersulfon, Angora, Schurwolle und/oder eine Kombination davon. Bevorzugt besteht das Tape aus Bauwollfasern. Des Weiteren bevorzugt umfasst das Tape 85% Nylon und 15% Spandex; 93% Nylon, 6% Polyethersulfon und 1% Elastan; 44% Angora und 26% Schurwolle; oder 26% Polyamid und 4% Elastan.

Weiterhin weist das eigentliche kinesiologische Tape in einem weiteren Beispiel, das nicht Teil der Erfindung ist, auf einer Seite eine am Körper haftende Schicht auf, wobei diese Schicht besonders bevorzugt ausgewählt wird aus der Gruppe, bestehend aus einer Acryl- und/oder Zinkoxid-Basis,

Die Form des eigentlichen kinesiologischen Tapes kann in einem weiteren Beispiel, das nicht Teil der Erfindung ist, unabhängig von der Form des mindestens einen Formkörpers und der aus mindestens zwei Formkörpern gebildeten Form ausgewählt werden. Die Form des eigentlichen Tapes wird bevorzugt ausgewählt aus der Gruppe, bestehend aus einem Dreieck, Kreis, Rechteck, Quadrat, Ellipse, Raute, Oval, Y-Form, gegebenenfalls ergänzt um weitere Linien, V-Form, S-Form, X-Form, C-Form, L-Form, T-Form, Kreuzform und/oder einem Rechteck mit mehreren Einschnitten, Besonders bevorzugt können auch mehrere kinesiologische Bänder verwendet werden, so dass sich eine der oben genannten Form durch das Zusammensetzen der einzelnen Tapes ergibt. Ein unterstützender Effekt kann auch dadurch erzielt werden, dass das Tape in unterschiedlich stark vorgedehnter Weise vorliegt.

Die Länge des eigentlichen kinesiologischen Tapes kann in einem weiteren Beispiel, das nicht Teil der Erfindung ist, an das jeweils zu behandelnde Körperteil angepasst werden. Weiterhin kann in einer Ausführungsofrm auch durch die Verwendung mehrerer kinesiologischer Bänder gleicher Länge eine Anpassung an das zu behandelnde Körperteil erfolgen, In einer anderen Ausführungsform werden mehrere kinesiologische Bänder unterschiedlicher Länge verwendet, In einer weiteren Ausführungsform werden mindestens drei kinesiologische Bänder verwendet, wobei mindestens zwei dieser Bänder die gleiche Länge aufweisen und mindestens ein Band eine Länge aufweist, die unterschiedlich von der Länge der anderen beiden Bänder ist,

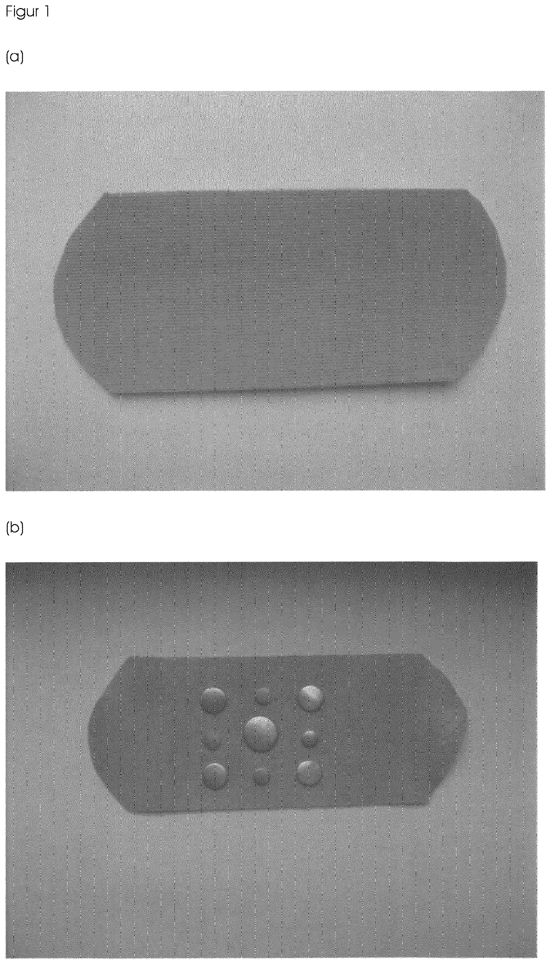

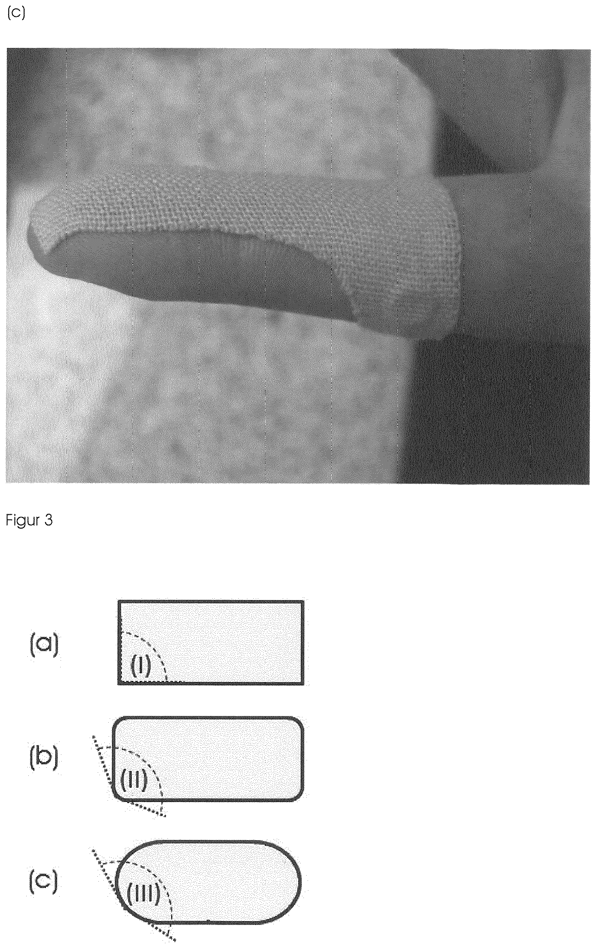

In einem weiteren Beispiel, das nicht Teil der Erfindung ist, weist das kinesiologische Tape keine Ecken mit einem Winkel von kleiner als 90° (zur Bestimmung des Winkels siehe Figur 3), bevorzugt kleiner als 100°, besonders bevorzugt kleiner als 110° und ganz besonders bevorzugt kleiner als 120° auf (siehe Figur 3 (b) und (c)), Besonders bevorzugt weist das Tape abgerundete Ecken auf (siehe Figur 1 und Figur 3(b) und (c)), Es hat sich herausgestellt, dass Tapes, die keine Ecken mit einem Winkel von kleiner als 90° aufweisen, weniger Angriffspunkte zum Ablösen aufweisen. Dadurch ist es möglich, dass das erfindungsgemäße kinesiologische Tape lange am Körper des Patienten haftet, in einem weiteren Beispiel, das nicht Teil der Erfindung ist, kann das kinesiologische Tape bevorzugt mindestens 14 Tage, besonders bevorzugt mindestens 10 Tage, ganz besonders bevorzugt mindestens 7 Tage am Körper des Patienten haften.

In einer besonders bevorzugten Ausführungsform wird durch den mindestens einen Formkörper des erfindungsgemäßen kinesiologischen Tapes punktuell Wärme auf den entsprechenden mindestens einen Reflexpunkt übertragen wird. Dadurch konnte gezeigt werden, dass zusätzlich zum synergistischen Effekt, der durch das erfindungsgemäße kinesiologische Tape mit mindestens einem Formkörper erzielt wird, eine Wärmetherapie effektiv umgesetzt werden kann, Durch den mindestens einen Formkörper kann die Wärme direkt auf den Reflexpunkt, der in einer Ausführungsform durch den mindestens einen Formkörper mit Druck beaufschlagt sein kann, übertragen werden. Da Kupfer als Material des mindestens einen Formkörpers verwendet wird, kann ein zusätzlicher heilender Effekt des erfindungsgemäßen kinesiologischen Tapes durch eine Wärmetherapie beobachtet werden. Die Wärme wird dabei von der vom Körper abgewandten Seite durch eine Vorrichtung ausgewählt aus der Gruppe, bestehend aus Infrarotstrahler, Wärmekissen, Wärmeflasche und/oder Kombinationen davon, auf den mindestens einen Reflexpunkt übertragen, Es hat sich als vorteilhaft herausgestellt, dass das erfindungsgemäße kinesiologische Tape derart ausgestaltet ist, dass der behandelte Patient selber durch Reibung der beklebten Körperstelle Wärme erzeugen kann, die durch den mindestens einen Formkörper auf den mindestens einen Reflexpunkt gezielt übertragen werden kann.

In einer weiteren Ausführungsform wird durch den mindestens einen Formkörper des erfindungsgemäßen kinesiologischen Tapes punktuell Kälte auf den entsprechenden mindestens einen Reflexpunkt übertragen wird. Dadurch konnte gezeigl werden, dass zusätzlich zum synergistischen Effekt, der durch das erfindungsgemäße kinesiologische Tape mit mindestens einem Formkörper erzielt wird, eine Kältetherapie effektiv umgesetzt werden kann. Durch den mindestens einen Formkörper kann die Kälte direkt auf den Reflexpunkt, der in einer Ausführungsform durch den mindestens einen Formkörper mit Druck beaufschlagt sein kann, übertragen werden. Da Kupfer als Material des mindestens einen Formkörpers verwendet wird, kann ein zusätzlicher heilender Effekt des erfindungsgemäßen kinesiologischen Tapes durch eine Kältetherapie beobachtet werden. Die Kälte wird dabei von der vom Körper abgewandten Seite durch eine Vorrichtung ausgewählt aus der Gruppe, bestehend aus Kältekissen, Eisbeutel, Kryogerät und/oder Kombinationen davon, auf den mindestens einen Reflexpunkt übertragen.

In einem weiteren Beispiel, das nicht Teil der Erfindung ist, werden durch den mindestens einen Formkörper des kinesiologischen Tapes punktuell Vibrationen auf den entsprechenden mindestens einen Reflexpunkt übertragen. Dadurch konnte gezeigt werden, dass zusätzlich zum synergistischen Effekt, der durch das erfindungsgemäße kinesiologische Tape mit mindestens einem Formkörper erzielt wird, der Abtransport von Schlagstoffen in der Muskulatur effektiv umgesetzt werden kann. Durch den mindestens einen Formkörper kann die Virbation direkt auf den Reflexpunkt, der in einer Ausführungsform durch den mindestens einen Formkörper mit Druck beaufschlagt sein kann, übertragen werden. Die Vibration wird dabei von der vom Körper abgewandten Seite durch eine Vorrichtung wie beispielsweise einem Vibrax auf den mindestens einen Reflexpunkt übertragen.

Zur weiteren Steigerung der Wirkung kann in einer weiteren Ausführungsform der mindestens eine Formkörper einen aufgetragenen Wirkstoff umfassen. Dieser Wirkstoff wird bevorzugt ausgewählt aus der Gruppe, bestehend aus entzündungshemmenden, durchblutungsfördernden, tonusregulierenden, schmerzstillenden, ionotophorestischen, trophikverbesserenden, ödemabflussfördernden, wärmeaufnehmenden Wirkstoffen und/oder eine Kombination davon. Besonders bevorzugt wird der Wirkstoff ausgewählt aus der Gruppe, bestehend aus Diclofenac, Ibuprofen, ätherischen Öle und homöopathischen Wirkstoffen wie Arnika und/oder Kombinationen davon.

Das erfindungsgemäße kinesiologisches Tape dient zur Behandlung von Krankheitsbildern, ausgewählt aus der Gruppe, bestehend aus HWS-Syndrom (Halswirbelsäulensyndrom), BWS-Syndrom (Brustwirbelsäulensyndrom), LWS-Syndrom (Lendenwirbelsäulensyndrom), Schulter-Arm-Syndrom, Bizeps-Sehnenreizung, Supraspinatussyndrom, Epicondylitis, Karpaltunnel-Syndrom, Craniomandibuläre Dysfunktion, Daumensattelgelenkschmerzen, Ganglion/Überbein, ISG-Syndrom, Ischialgie, Lumboischialgie, Coxarthrose, Piriformis-Syndrom, Gonarthrose, Distorsion, Spastikhemmung, Myogelose/physikalische Myalgie, Parese, Ödeme, Morbus Bechterew, Fersensporn, Sportverletzungen wie Muskelzerrungen, Muskelkater, Achillodynia und/oder Muskelfaserriss, Schmerzen wie Gelenkschmerzen, Gelenkergüsse, Kopfschmerzen, Ellenbogenschmerzen, Schulterschmerzen, Handgelenkschmerzen, Rückenschmerzen im oberen (HWS), mittleren (BWS) und/oder unteren Rücken (LWS), Steißbeinschmerzen, Hüftschmerzen, Knieschmerzen, Schmerzen im Bereich der Achillessehne, Sprunggelenkschmerzen, Schmerzen im Bereich der Ferse, Schmerzen in Folge von Totalendoprothesen (TEP), insbesondere in der Hüfte oder im Knie, internistische Erkrankungen wie Verdauungsstörungen, Bauchschmerzen, chronischer Verstopfung und/oder Kombinationen davon.

Besonders bevorzugt werden die oben genannten Krankheitsbilder mit dem erfindungsgemäßen kinesiologischen Tape bei erkrankten Menschen behandelt.

In einem weiteren Beispiel, das nicht Teil der Erfindung ist, kann das erfindungsgemäße kinesiologische Tape bevorzugt durch eine zusätzliche Fixierung am zu behandelnden Körper aufgebracht werden. Solch eine Fixierung kann bevorzugt die Haftungseigenschaften des erfindungsgemäßen kinesiologischen Tapes am Körper unterstützen und verbessern. Dadurch ist es möglich, dass das erfindungsgemäße kinesiologische Tape bevorzugt mindestens 14 Tage, besonders bevorzugt mindestens 10 Tage, ganz besonders bevorzugt mindestens 7 Tage am Körper des Patienten haftet.

Eine solche zusätzliche Fixierung stellt vorzugsweise eine Bandage oder Verband dar. Diese Bandage kann vorzugsweise elastische Materialien umfassen. Besonders bevorzugt umfasst die Bandage Materialien, ausgewählt aus der Gruppe, bestehend aus duroplastischen Kunststoffen, thermoplastischen Kunststoffen, Elastomeren, Epoxidharzen, Kautschuk, Polyesterharzen, Silikonen, Kunststofffasern, Aramidfasern, Glasfasern, Nylon, Perlon, Baumwolle und Elastan.

Die zusätzliche Fixierung kann auf einem kinesiologische Tape aufgebracht werden.

In einem weiteren Beispiel, das nicht Teil der Erfindung ist, weist die zusätzliche Fixierung auf einer Seite eine Klebeschicht auf. Diese Klebeschicht kann vorzugsweise dazu genutzt werden, dass sie auf dem kinesiologischen Tape und/oder der Haut des Patienten haftet und dadurch eine zustätzliche Fixierung gewährleistet. Dabei umfasst die Klebeschicht besonders bevorzugt die oben genannten Materialien für Klebeschichten.

In einem weiteren Beispiel, das nicht Teil der Erfindung ist, ist es auch möglich, dass die Klebeschicht nur auf einen Teil der zusätzlichen Fixierung aufgebracht ist. Hierbei wird die zusätzliche Fixierung bevorzugt so auf das erfindungsgemäße kinesiologische Tape aufgebracht, dass es von der zusätzlichen Fixierung umschlossen wird, Die Fixierung selbst wird dabei bevorzugt durch den Teil, der die Klebeschicht aufweist mit einem anderen Teil der zusätzlichen Fixierung verbunden. Dies Beispiel ist besonders geeignet für die Anwendung an Körperteilen, die durch die Fixierung umschlossen werden können. Solche Körperteile können bevorzugt ausgewählt werden aus der Gruppe, bestehend aus Handgelenk, Ellenbogen, Oberarm, Unterarm, Rumpf, Kopf, Oberschenkel, Knie, Unterschenkel, Knöchel und Fuß. Dazu weist die zusätzliche Fixierung bevorzugt eine entsprechend angepasste Form auf. Es ist beispielsweise möglich, dass die Fixierung insgesamt die gleichen Maße aufweist wie das erfindungsgemäße kinesiologische Tape, jedoch ein wenig länger ist. Auf diesem längeren Teil befindet sich vorzugsweise die Klebeschicht. Vorzugsweise ist die Stelle, an der die Klebeschicht aufgebracht wird, schmaler als der Rest der Fixierung. Bevorzugt ist der Bereich der zusätzlichen Fixierung, der mit einer Klebeschicht versehen ist 0,2 bis 20 cm, besonders bevorzugt 0,4 bis 17 cm, ganz besonders bevorzugt 0,5 bis 14 cm. Der Vorteil dieser Ausführungsform besteht darin, dass die Klebeschicht im Wesentlichen auf der anderen Seite der zusätzlichen Fixierung aufgebracht wird (beispielsweise beim Umschließen eines Handgelenkes). Dadurch können für diese Klebeschicht auch Materialien verwendet werden, die eine strake Haftwirkung, jedoch gegebenenfalls eine allergische Wirkung haben. Da kein Kontakt mit der Haut des Patienten besteht, können allergische Reaktionen gegen diesen Klebstoff, aber gleichzeitig eine stark Haftung realisiert werden.

In einem weiteren Beispiel, das nicht Teil der Erfindung ist, ist die zusätzliche Fixierung an einer Stelle anstatt mit einer Klebeschicht oder zusätzlich zu dieser mit mindestens einem Clip oder Knopf ausgestattet. Besonders bevorzugt wird hier keine Klebeschicht verwendet, wodurch allergische Reaktionen gegen die Bestandteile der Klebeschicht vermieden werden können, aber dennoch eine feste Fixierung durch den mindestens einen Clip oder Knopf garantiert wird.

Ein besonderer Vorteil des erfindungsgemäßen kinesiologischen Tapes ergibt sich gerade durch die oben beschriebenen flexiblen Ausgestaltungsformen. Es ist möglich, durch das erfindungsgemäße kinesiologische Tape die Form bzw. unterschiedlichen Formen des mindestens einen Formkörpers sowie auch des eigentlichen Tapes individuell auf das Krankheitsbild eines einzelnen Patienten anzupassen, sowie auch auf die individuellen Körperformen des jeweiligen Patienten,

In einem weiteren Aspekt betrifft die Erfindung ein Kit of parts, umfassend ein kinesiologisches Tape und mindestens einen Formkörper zur Druckausübung mit Akupressurwirkung auf eines beklebte Körperteil, der ein Material umfasst, welches Kupfer ist. In einer bevorzugten Ausführungsform kann dieses Kit of parts zur Behandlung der oben genannten Krankheitsbilder verwendet werden. Dieses Kit of parts umfasst mindestens einen Formkörper, wie er oben beschrieben ist, sowie ein wie oben beschriebenes kinesiologisches Tape.

Die individuelle Anpassung kann an den folgenden Ausführungsbeispielen erläutert werden:
Zur Behandlung von Spastikehmmung wird das erfindungsgemäße kinesiologische Tape bevorzugt mit spitzen Formkörpern eingesetzt.

Zur Behandlung von Ödemen wird das erfindungsgemäße kinesiologische Tape bevorzugt mit mindestens einem Formkörper mit einem Durchmesser von bevorzugt 0,1 bis 0,5 cm, besonders bevorzugt 0,15 bis 0,2 cm eingesetzt,

Zur Behandlung von Fersensporn wird das erfindungsgemäße kineslologische Tape bevorzugt mit mindestens einem konkaven Formkörper eingesetzt, wobei der Fersensporn durch diese Form des Formkörpers umhüllt wird. In einer weiteren Ausführungsform werden zur Behandlung von Fersensporn auf das erfindungsgemäße kinesioloische Tape bevorzugt mehrere Formkörper in einem Kreis (O-Form) aufgebracht, wobei das Tape so aufgebracht wird, dass der Fernsonsporn sich in der von den Formkörpern umschlossenen Fläche befindet.

Zur Entlastung des Kaumuskels und des Kiefergelenkes bei Craniomandibulärer Dysfunktion durch Aufbringung am Kiefergelenk wird das erfindungsgemäße kinesiologische Tape bevorzugt mit mindestens einem runden, flachen oder leicht gewölbten Formkörper verwendet. Der Formkörper weist vorzugsweise einen Durchmesser von 0,3 bis 1,0 cm, bevorzugt 0,4 bis 0,9 cm, besonders bevorzugt 0,6 bis 0,8 cm auf. Die Tapelänge beträgt bevorzugt 0,5 bis 20 cm. Es ist aber auch möglich, das Tape am Kiefergelenk so aufzubringen, dass mehrere Tapestreifen hintereinander aufgeklebt werden, Das Tape ist bevorzugt rechteckig und/oder rund.

Zur Behandlung von Beschwerden im Kopfbereich durch Aufbringung an den Schläfen, zwischen den Augenbrauen, am Augenlid und/oder an der Stirn wird mindestens ein runder, flacher oder leicht gewölbter Formkörper verwendet, Der Formkörper weist vorzugsweise einen Durchmesser von 0,3 bis 1,0 cm, bevorzugt 0,4 bis 0,9 cm, besonders bevorzugt 0,6 bis 0,8 cm auf, Die Tapelänge beträgt vorzugsweise 0,5 bis 20 cm. Die Tapeform ist bei der Aufbringung auf die Schläfen, Stirn oder zwischen den Augenlidern bevorzug rechteckig. Zur Aufbringung am Augenlid wird bevorzugt ein C-förmiges Tape gewählt.

Zur Behandlung des HWS-Syndroms bei Kopfschmerzen, Schwindel, Tinnitus, Augendruck und/oder ziehenden Schmerzen in den Armen durch Aufbringung am Schiefhals wird das kinesiologische Tape bevorzugt am Hinterkopf in Richtung Schultern und/oder neben oder auf der HWS aufgebracht. Dabei weist der mindestens eine Formkörper vorzugsweise eine Dicke von 0,5 bis 2,5 mm Dicke auf. Die Tapelänge beträgt vorzugsweise 15 bis 30 cm. Dabei wird bevorzugt eine Tapefrom in Form eines Y oder einer geraden Linie gewählt. Besonders bevorzugt wird ein gerades Tape mit zwei parallel verlaufenden Linien von mehreren Formkörpern gewählt, wobei die Linien der Formkörper links und rechts von der HWS verlaufen.

Zur Behandlung des BWS-Syndroms bei Atemnot und/oder ausstrahlenden Schmerzen wird das kinesiologische Tape bevorzugt neben oder auf der BWS aufgebracht. Die Tapelänge beträgt bevorzugt 10 bis 40 cm. Hierbei können bevorzugt zwei erfindungsgemäße im Wesentlichen gerade Tapes mit mindestens drei im Wesentlichen linear aufgebrachten Formkörpern parallel zur BWS aufgebracht werden, In einer anderen Ausführungsform wird nur ein Tape verwendet, welches beide parallel zur BWS verlaufenden Linien der Formkörper aufweist. Bevorzugt können zusätzlich lineare erfindungsgemäße kinesiologische Tapes von den Schultern gerade bis zur BWS aufgebracht werden. Diese können optional mindestens drei Formkörper in linearer Anordnung aufweisen. In einer weiteren Ausführungsform ist sowohl die Tapeform, sowie auch die Form, die durch die Formkörper gebildet wird S-förmig und/oder spiegelverkehrt und wird neben der BWS aufgebracht.

Zur Behandlung des LWS-Syndroms bei Schmerzen, ausstrahlenden Schmerzen, Bandscheibenproblemen und/oder degenerativem Verschleiß der Wirbelgelenke wird das erfindungsgemäße kinesiologische Tape bevorzugt T- und/oder C-förmig im Bereich der LWS aufgebracht. In einer Ausführungsform werden die entsprechenden Formen neben der LWS aufgebracht. In einer weiteren Ausführungsform können im Wesentlichen lineare erfindungsgemäße kinesiologische Tapes horizontal über den LWS Bereich oder rechts und links von der LWS aufgebracht werden.

Zur Behandlung von Kniearthrosen, Sportverletzungen am Knie, traumatischen Verletzungen am Knie und/oder postoperativen Behandlungen am Knie wird das erfindungsgemäße kinesiologische Tape bevorzugt mit einem Formkörper mit einem Durchmesser von 10 cm aufgebracht. Die Aufbringung erfolgt dabei bevorzugt mit zwei erfindungsgemäßen kinesiologischen Tapes jeweils lateral und medial am Knie.

Zur Behandlung von Paresen wird das erfindungsgemäße kinesiologische Tape bevorzugt mit mindestens einem Formkörper mit einem Durchmesser von bevorzugt 0,05 bis 0,5 cm, besonders bevorzugt 0,15 bis 0,2 cm und/oder mindestens einem spitzen Formkörper aufgebracht. Die Tapelänge beträgt vorzugsweise 30 bis 50 cm.

Zur Behandlung des Karpaltunnel-Syndroms wird das erfindungsgemäße kinesiologische Tape in einer Abfolge von zwei oder mehr runden Formkörpern gleicher oder unterschiedlicher Größe aufgebracht. Die Tapelänge beträgt bevorzugt 1 bis 10 cm, besonders bevorzugt 3 bis 7 cm.

Zur Behandlung von Ganglion wird das erfindungsgemäße kinesiologische Tape bevorzugt mit mindestens einem Formkörper mit 1,5 cm Durchmesser aufgebracht, Der Formkörper weist bevorzugt eine Dicke von 0,5 cm auf. Die Tapelänge beträgt vorzugsweise 5 cm.

Zur Behandlung von Schmerzen im Armbereich wird das erfindungsgemäße kinesiologische Tape bevorzugt im Oberarmbereich aufgebracht. Das Tape kann vorzugsweise in einer L-Form, einer S-Form oder in mehreren parallel angeordneten im Wesentlichen geraden Linien gleicher oder unterschiedlicher Größe aufgebracht sein. Zur Unterstützung der Heilung von Narbengewebe wird das erfindungsgemäße kinesiologische Tape bevorzugt um das Narbengewebe aufgebracht. Dabei wird die Narbe an sich vorzugsweise nicht beklebt, das bedeutet, das erfindungsgemäße kinesiologische Tape wird um das betroffene Narbengewebe herum aufgebracht.

Bevorzugte Ausführungsformen sind der Tabelle 1 zusammengefasst.

**Tabelle 1: mögliche Ausführungsform der Erfindung**

| **Tapeform** | **Formkörper-Form** | **Form des Formkörpers/der Formkörper auf dem Tape** | **Krankheitsbild** | **Position auf dem Körper** |
|---|---|---|---|---|
| rechteckig | rund, leicht gewölbt | 1 mittig aufgebrachter Formkörper | Schwindel, Kopfschmerzen, Migräne, Tinnitus | Stirn |
| rechteckig | rund, leicht gewölbt | 1 mittig aufgebrachter Formkörper | Kopfschmerzen | zwischen Augenbrauen |
| rechteckig | halbkugelförmig | 1 mittig aufgebrachter Formkörper | Kopfschmerzen | zwischen Augenbrauen |
| rund | halbkugelförmig | 1 mittig aufgebrachter Formkörper | Kopfschmerzen | zwischen Augenbrauen |
| rund | rund, leicht gewölbt | 1 mittig aufgebrachter Formkörper | Kopfschmerzen | zwischen Augenbrauen |
| rechteckig | rund, leicht gewölbt | 1 mittig aufgebrachter Formkörper | Craniomandibuläre Dysfunktion | Kiefergelenk |
| rechteckig | rund, leicht gewölbt | mehrere im Wesentlichen linear aufgebrachte Formkörper | Craniomandibuläre Dysfunktion | Kiefergelenk |
| rechteckig | rund, leicht gewölbt | 1 mittig aufgebrachter Formkörper | Kopfschmerzen, Migräne | Schläfe |
| rechteckig | halbkugelförmig | 1 mittig aufgebrachter Formkörper | Kopfschmerzen, Migräne | Schläfe |
| rund | rund, leicht gewölbt | 1 mittig aufgebrachter Formkörper | Kopfschmerzen, Migräne | Schläfe |
| rund | rund, leicht gewölbt | 1 mittig aufgebrachter Formkörper | Craniomandibuläre Dysfunktion | Kiefergelenk |
| C-förmig | rund, leicht gewölbt | C-förmige Anordnung mehrere Formkörper | Augenringe, Lymphstau | Augenlid |
| im Wesentlichen linear | rund, leicht gewölbt | mehrere im Wesentlichen linear aufgebrachte Formkörper | Schwindel, Kopfschmerzen | Verlauf: hinter einem Ohr über Hals bis zum Brustbein |
| Y-förmig | rund, leicht gewölbt | mehrere y-förmig aufgebrachte Formkörper | Schwindel, Kopfschmerz | Verlauf: hinter einem Ohr über Hals |
| im Wesentlichen linear | rund, leicht gewölbt | mehrere Formkörper in mehreren parallel zu einander verlaufenden Linien | HWS-Syndrom und entsprechende Symptome | auf HWS (Formkörperlinien verlaufen neben Wirbelkörpern) |
| im Wesentlichen linear | rund, leicht gewölbt | mehrere Formkörper in Linie | HWS-Syndrom und entsprechende Symptome | Verlauf: von Hals bis zur Schulter |
| im Wesentlichen linear | rund, leicht gewölbt | mehrere Formkörper in Linie | HWS-Syndrom und entsprechende Symptome | Umgreifen der Schulter |
| drei im Wesentlichen linear verlaufende Tapes, die zusammen eine Y-Form ergeben | rund, leicht gewölbt | mehrere Formkörper in mehreren parallel zu einander verlaufenden Linien | BWS-Syndrom und entsprechende Symptome | 1 Tape senkrecht im BWS-Bereich (Formkörperlinien verlaufen neben Wirbelkörpern); jeweils 2 Tapes von Schultern zu dem mittig angebrachten Tape |
| Zwei S-förmige bzw. spiegelverkehrt S-förmige Tapes | rund, leicht gewölbt | S-förmige bzw. spiegelverkehrt S-förmige Anordnung | BWS-Syndrom und entsprechende Symptome | seitlich versetzt rechts und links von der Wirkbelsäule |
| C-förmig | rund, leicht gewölbt | C-förmig | LWS-Syndrom und entsprechende Symptome | seitlich versetzt zur LWS |
| T-förmig | rund, leicht gewölbt | T-förmig | LWS-Syndrom und entsprechende Symptome | seitlich versetzt zur LWS |
| im Wesentlichen linear | rund, leicht gewölbt | mehrere Formkörper in Linie | LWS-Syndrom und entsprechende Symptome | horizontal über LWS hinweg |
| 2 im Wesentlichen lineare Tapes | rund, leicht gewölbt | mehrere Formkörper in Linie | LWS-Syndrom und entsprechende Symptome | links und rechts neben der LWS |
| 2 im Wesentlichen lineare Tapes, die zusammen eine V-Form ergeben | rund, leicht gewölbt | V-förmig | Achillodynia | hinterer, oberer Wadenbereich (Öffnung des V) bis zur Ferse |
| rund | rund, leicht gewölbt | 1 mittig aufgebrachter Formkörper | Tennisarm (außen), Golferarm (innen) | Unterarm |
| rechteckig | oval | 1 mittig aufgebrachter Formkörper | Arthrose, nach Operationen oder Trauma | seitliches Kniegelenk |
| mehrere im Wesentlichen linear verlaufende Tapes, die zusammen eine Y-Form mit zusätzlich mittig verlaufenden Schenkel ergeben | rund, leicht gewölbt | Y-förmig mit zusätzlich mittig verlaufenden Schenkel | Lumboischialgie, Sportverletzungen und Symptome | hinterer, oberer Oberschenkel (Öffnung des Y) bis hin zum Kniegelenk |
| rund | rund | kreisförmig | Fersensporn | Ferse |
| T-förmig | rund | 2 Formkörper jeweils an den Flügeln des T | Arthritis | Finger |
| T-förmig | rund | 2 Formkörper jeweils an den Flügeln des T | Karpaltunnelsyndrom | Handgelenk |
| T-förmig | rund | 2 Formkörper jeweils an den Flügeln des T | Sportverletzungen | Fußgelenk |

### Kurze Beschreibung der Figuren

Figur 1: Abbildung einer bevorzugten Ausführungsform des erfindungsgemäßen kinesiologischen Tapes mit Ecken, welche alle größer als 90° sind (Länge des Tapes in der Mitte: 15 cm)
   (a) Aufnahme des kinesiologischen Tapes von der zu beklebenden Körperstelle abgewandten Seite;
   (b) Aufnahme des kinesiologischen Tapes von der zu beklebenden Körperstelle zugewandten Seite (Anordung der Formkörper aus Kupfer in einem Rechteck, wobei die Formkörper einen Durchmesser aufweisen von (obere und untere Reihe von links nach rechts): 0,8 cm; 0,5 cm; 0,8 cm; (mittlere Reihe von links nach rechts): 0,5 cm; 1,1 cm; 0,5 cm)

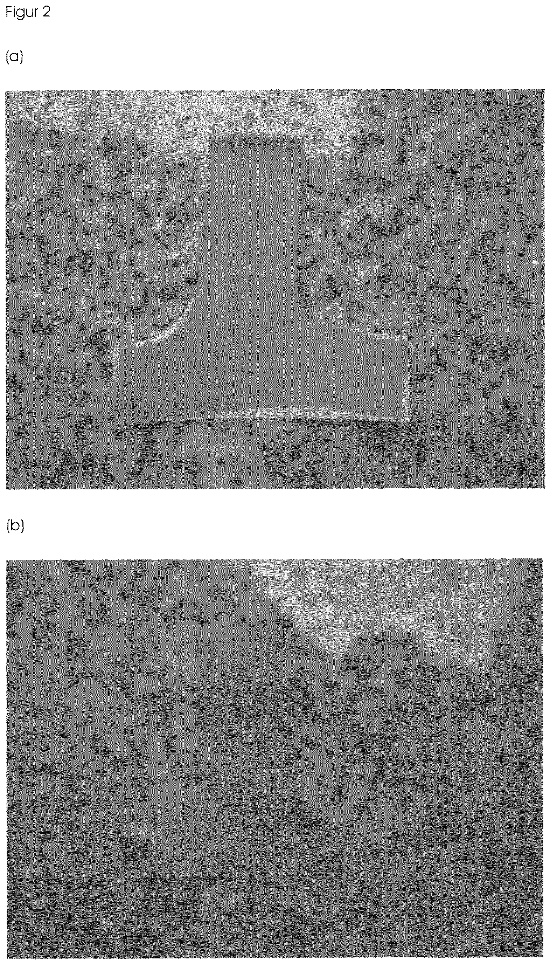

Figur 2: Abbildung einer bevorzugten Ausführungsform des erfindungsgemäßen kinesiologischen Tapes zur Anbringung an Finger, Hand- oder Fußgelenke, wobei die Tapeform einem T entspricht (je nach Anwendung beträgt die Tapelänge 3 bis 10 cm, sowie auch die Breite (Flügel) 3 bis 10 cm)
   (a) Aufnahme des kinesiologischen Tapes von der zu beklebenden Körperstelle abgewandten Seite,
   (b) Aufnahme des kinesiologischen Tapes von der zu beklebenden Körperstelle zugewandten Seite (Anbringung jeweils eines Formkörpers aus Kupfer an den Flügeln; Durchmesser der Formkörper: 0,5 cm)
   (c) Anbringung des kinesiologischen Tapes an einem Finger
Figur 3: schematische Darstellung des Winkels der Ecken des erfindungsgemäßen kinesiologischen Tapes durch Anlegen von Tangenten
   (a) kinesiologisches Tape, wobei die Ecken einen Winkel von 90° (I) aufweisen
   (b) kinesiologisches Tape, wobei die Ecken einen Winkel von größer 90° (II) aufweisen (abgerundete Ecken)
   (c) kinesiologisches Tape, wobei die Ecken einen Winkel von größer 120° (III) aufweisen (abegrundete Ecken)

### Beispiele

In der folgenden Tabelle 2 werden beispielhaft die Ergebnisse der Anwendung des erfindungsgemäßen kinesiologischen Tapes in der Schmerztherapie unterschiedlicher Krankheitsbilder aufgeführt. Dazu beurteilten die Patienten zunächst in einer Skala von 0 bis 10 ihren Schmerz vor Beginn der Behandlung. Dabei bedeutet ein Wert von 0 keine Schmerzen und ein Wert von 10 sehr starke Schmerzen, Daraufhin wurden die Patienten mit dem erfindungsgemäßen kinesiologischen Tape behandelt. Nach 7 Tagen nach Beginn der Behandlung beurteilten die jeweiligen Patienten nochmals mittels der oben genannten Skala von 0 bis 10 ihre Schmerzen. In dieser Zeitspanne von 7 Tagen wurde das erfindungsgemäße kinesiologische Tape nicht erneuert.

**Tabelle 2: Ergebnisse der Schmerztherapie unterschiedlicher Krankheitsbilder mit dem erfindungsgemäßen kinesiologischen Tape**

| **Patient** | **Datum der Behandlung** | **Geschlecht des Patienten** | **Geburtsdatum des Patienten** | **Krankheitsbild** | **Beurteilung Schmerz vor Beginn der Behandlung** | **Beurteilung Schmerz nach 7 Tagen** |
|---|---|---|---|---|---|---|
| 1 (Mensch) | 30.08.2011 | Männlich | 09.10.1942 | HWS-Syndrom nach Unfall | 7 | 3 |
| 2 (Mensch) | 30.08.2011 | Weiblich | 27.07.1944 | LWS-Syndrom | 9 | 1 |
| 3 (Mensch) | 06.09.2011 | Weiblich | 02.09.1939 | Hüfte TEP rechts | 10 | 0 |
| 4 (Mensch) | 22.08.2011 | Weiblich | 26.09.1948 | Bandscheibenvorwölbung | 5 | 0 |
| 5 (Mensch) | 22.08.2011 | Männlich | 05.10.1958 | Knie-OP | 7 | 5 |
| 6 (Mensch) | 02.08.2011 | Weiblich | 06.09.1961 | Bandscheibenvorfall LWS | 8 | 3 |
| 7 (Mensch) | 18.08.2011 | Weiblich | 15.11.2000 | Knieschmerzen | 8 | 1 |
| 8 (Mensch) | 18.08.2011 | Männlich | 03.01.1944 | Tennisellenbogen | 7 | 0 |
| 9 (Mensch) | 22.08.2011 | Männlich | 10.11.1941 | Apoplex re. Arm Spastik | 8 | 3 |
| 10 (Mensch) | 27.08.2011 | Weiblich | 19.05.1954 | Banscheibenvorfall | 6 | 2 |
| 11 (Mensch) | 23.08.2011 | Weiblich | 25.04.1975 | LWS-Syndrom | 7 | 0 |
| 12 (Mensch) | 25.08.2011 | Männlich | 19.08.1939 | Schulter-Armsyndrom | 6 | 4 |
| 13 (Mensch) | 25.08.2011 | Weiblich | 01.07.1987 | LWS-Syndrom | 10 | 0 |
| 14 (Mensch) | 25.08.2011 | Männlich | 10.04.1963 | HWS-Syndrom | 2 | 0 |
| 15 (Mensch) | 01.09.2011 | Männlich | 15.06.1934 | LWS-Syndrom nach ca. 5 Jahren | 8 | 1 |
| 16 (Mensch) | 29.08.2011 | Weiblich | 23.12.1952 | Banscheibenvorwölbung | 8 | 0 |
| 17 (Mensch) | 22.08.2011 | Männlich | 03.02.1974 | LWS-Syndrom | 8 | 1 |
| 18 (Pferd) | 02.09.2011 | Weiblich | | Konnte schlecht laufen | - | Konnte wieder laufen |
| 19 (Mensch) | 02.09.2011 | Weiblich | 05.11.1976 | Kribbeln in Fingern rechts, Arm | 8 | 0 |
| 20 (Mensch) | 02.09.2011 | Weiblich | 08.02.1939 | Knie TEP links | 5 | 0 |

## Patentansprüche

1. Kinesiologisches Tape mit an den menschlichen Muskel angepassten Elastizitätseigenschaften, **dadurch gekennzeichnet, dass** das Tape mindestens einem Formkörper zur Druckausübung mit Akupressurwirkung auf eines beklebte Körperteil aufweist, der ein Material umfasst, welches Kupfer ist.

2. Kinesiologisches Tape gemäß Anspruch 1, **dadurch gekennzeichnet, dass** der mindestens eine Formkörper derart ist, dass er einen flächigen und/oder punktuellen Druck auf mindestens einen Reflexpunkt ausüben kann.

3. Kinesiologisches Tape gemäß einem der Ansprüche 1 bis 2, **dadurch gekennzeichnet, dass** der mindestens eine Formkörper eine Form besitzt, ausgewählt aus der Gruppe, bestehend aus Kugel, Kugelkette, Ring, Halbkugel, Ellipsoid, Rotationsellipsoid, Paraboloid, Rotationsparaboloid, Hyperboloid, Oloid, Polyeder, Tetraeder, Würfel, Quader, Oktaeder, Ikosaeder, Dodekaeder, Kuboktaeder, Rhombendodekaeder, Ikosidodekaeder, Rhombentriaktontaeder, Deltaeder, Pyramide, Prisma, Antiprisma, Pyramidenstumpf, Torus, Zylinder, Kegel, Kegelstumpf, geodätischer Kuppel und/oder Kombinationen davon.

4. Kinesiologisches Tape gemäß einem der Ansprüche 1 bis 3, **dadurch gekennzeichnet, dass** durch den mindestens einen Formkörper punktuell Wärme und/oder Kälte auf den entsprechenden mindestens einen Reflexpunkt übertragen wird.

5. Kinesiologisches Tape gemäß einem der Ansprüche 1 bis 4, **dadurch gekennzeichnet, dass** der Formkörper einen aufgetragenen Wirkstoff ausgewählt aus der Gruppe, bestehend aus entzündungshemmenden, durchblutungsfördernden, tonusregulierenden, schmerzstillenden, ionotophorestischen, trophikverbesserenden, ödemabflussfördernden, wärmeaufnehmende Wirkstoffen und/oder eine Kombination davon umfasst.

6. Kit of parts umfassend ein kinesiologisches Tape mit an den menschlichen Körper angepassten Elastitzitätseigenschaften, **dadurch gekennzeichnet, dass** das Tape mindestens einen Formkörper zur Druckausübung mit Akupressurwirkung auf eines beklebte Körperteil aufweist, der ein Material umfasst, welches Kupfer ist.

7. Kit of parts gemäß Anspruch 6, umfassend mindestens einen Formkörper gemäß einem der Ansprüche 2 bis 5.

## Claims

1. A kinesiologic tape having elasticity properties adapted to the human muscle, **characterized in that** the tape comprises at least one moulded body for applying pressure with acupressure impact onto a part of the body covered with the tape, which moulded body comprises a material which is copper.

2. A kinesiologic tape according to claim 1, **characterized in that** the at least one moulded body is configured such that it can apply a surface pressure and/or a targeted pressure to at least one reflex point.

3. A kinesiologic tape according to one of the claims 1 through 2, **characterized in that** the at least one moulded body comprises a shape which is selected from the group consisting of a sphere, a ball chain, a ring, a hemisphere, an ellipsoid, a spheroid, a paraboloid, a rotational paraboloid, a hyperboloid, an oloid, a polyhedron, a tetrahedron, a cube, a cuboid, an octahedron, an icosahedron, a dodecahedron, a cuboctahedron, a rhombic dodecahedron, an icosidodecahedron, a rhombic triacontahedron, a deltahedron, a pyramid, a prism, an antiprism, a frustum of pyramid, a torus, a cylinder, a cone, a truncated cone, a geodesic dome and/or combinations thereof.

4. A kinesiologic tape according to one of the claims 1 through 3, **characterized in that** thanks to the at least one moulded body, heat and/or coldness will be selectively transferred to the corresponding at least one reflex point.

5. A kinesiologic tape according to one of the claims 1 through 4, **characterized in that** the moulded body comprises an applied active substance selected from the group consisting of anti-inflammatory substances, substances that stimulate the blood circulation, tonus-regulating substances, analgesic substances, ionotophorestic substances, trophies improving substances, substances that stimulate the outflow of edemas, heat absorbing substances and/or a combination thereof.

6. A kit of parts comprising a kinesiologic tape having elasticity properties adapted to the human body, **characterized in that** the tape comprises at least one moulded body for applying pressure with acupressure impact onto a part of the body covered with the tape, which moulded body comprises a material which is copper.

7. A kit of parts according to claim 6, comprising at least one moulded body according to one of the claims 2 through 5.

## Revendications

1. Ruban adhésif kinésiologique ayant des propriétés élastiques adaptées au muscle humain, **caractérisé en ce que** le ruban adhésif comprend au moins un corps moulé pour exercer une pression avec un effet d'acupression sur une partie du corps couverte par le ruban adhésif, le corps moulé comprenant un matériau qui est du cuivre.

2. Ruban adhésif kinésiologique selon la revendication 1, **caractérisé en ce que** l'au moins un corps moulé est configuré de sorte qu'il peut exercer une pression plane et/ou ponctuelle sur au moins un point réflexe.

3. Ruban adhésif kinésiologique selon l'une des revendications 1 à 2, **caractérisé en ce que** l'au moins un corps moulé comprend une forme sélectionnée dans le groupe composé d'un globe, d'une chaîne à billes, d'un anneau, d'une hémisphère, d'un ellipsoïde, d'un ellipsoïde de révolution, d'un paraboloïde, d'un paraboloïde de révolution, d'un hyperboloïde, d'un oloïde, d'un polyèdre, d'un tétraèdre, d'un cube, d'un parallélépipède, d'un octaèdre, d'un icosaèdre, d'un dodécaèdre, d'un cuboctaèdre, d'un dodécaèdre rhombique, d'un icosidodécaèdre, d'un triacontaèdre rhombique, d'un deltaèdre, d'un pyramide, d'un prisme, d'un antiprisme, d'un tronc de pyramide, d'un tore, d'un cylindre, d'un cône, d'un cône tronqué, d'une coupole géodésique et/ou des combinaisons de ceux-ci.

4. Ruban adhésif kinésiologique selon l'une des revendications 1 à 3, **caractérisé en ce que** grâce à l'au moins un corps moulé de la chaleur et/ou de la froideur est transmise sur l'au moins un point réflexe correspondant.

5. Ruban adhésif kinésiologique selon l'une des revendications 1 à 4, **caractérisé en ce que** le corps moulé comprend un agent actif appliqué, qui est sélectionné dans le groupe composé d'agents anti-inflammatoires, d'agents stimulant la circulation sanguine, d'agents visant à réguler le tonus, d'agents analgésiques, d'agents ionotophorestiques, d'agents améliorant la trophicité, d'argents stimulant le drainage d'oedèmes, d'argents absorbant la chaleur et/ou d'une combinaison de ceux-ci.

6. Kit d'éléments comprenant un ruban adhésif kinésiologique ayant des propriétés élastiques adaptées au corps humain, **caractérisé en ce que** le ruban adhésif comprend au moins un corps moulé pour exercer une pression avec un effet d'acupression sur une partie du corps couverte par le ruban adhésif, le corps moulé comprenant un matériau qui est du cuivre.

7. Kit d'éléments selon la revendication 6, comprenant au moins un corps moulé selon l'une des revendications 2 à 5.
